# EUROPÄISCHE PATENTSCHRIFT

(11) **EP 1 078 072 B1**
(45) Veröffentlichungstag und Bekanntmachung des Hinweises auf die Patenterteilung: **17.01.2007**
(21) Anmeldenummer: 99925017.8
(22) Anmeldetag: 20.05.1999
(51) Int. Cl.: C12N 15/55, C12N 15/62, C07K 1/22, G01N 33/53

(54) **NEUE PEPTIDFRAGMENTE ZUR REINIGUNG VON PROTEINEN**
NEW PEPTIDE FRAGMENTS FOR PROTEIN PURIFICATION
NOUVEAUX FRAGMENTS PEPTIDIQUES POUR LA PURIFICATION DE PROTEINES

(30) Priorität: 20.05.1998 DE 19822823
(43) Veröffentlichungstag der Anmeldung: 28.02.2001
(73) Patentinhaber: BASF Aktiengesellschaft, 67056 Ludwigshafen (DE)
(72) Erfinder: HAUER, Bernhard, D-67136 Fu gönheim (DE); SCHMID, Rolf, D., D-70569 Stuttgart (DE); ENZELBERGER, Markus, D-70569 Stuttgart (DE); MINNING, Stephan, D-70569 Stuttgart (DE)
(86) Internationale Anmeldenummer: PCT/EP1999/003469
(87) Internationale Veröffentlichungsnummer: WO 1999/060134

(56) Entgegenhaltungen:
- EP-A- 0 282 042
- EP-A- 0 409 814
- VOLZ J ET AL: "Molecular characterization of metal -binding polypeptide domains by electrospray ionization mass spectrometry and metal chelate affinity chromatography." JOURNAL OF CHROMATOGRAPHY. A, (1998 MAR 20) 800 (1) 29-37. , XP004112384 in der Anmeldung erwähnt
- MELCHERS K ET AL: "Cloning and membrane topology of a P type ATPase from Helicobacter pylori." JOURNAL OF BIOLOGICAL CHEMISTRY, (1996 JAN 5) 271 (1) 446-57. , XP002118565

## Beschreibung

Die Erfindung betrifft neue Peptidfragmente, Fusionsproteine enthaltend die Peptidfragmente, Verfahren zur ihrer Herstellung sowie die Verwendung der Peptidfragmente. Die Erfindung betrifft auch ein Verfahren zur Reinigung der Fusionsproteine und ein Verfahren zur Detektion von Proteinen.

Weiterhin betrifft die Erfindung Nukleinsäuren, die für die Peptidfragmente oder für die Fusionsproteine kodieren und Vektoren, die die Nukleinsäuren enthalten.

Durch die moderne Molekularbiologie ist es möglich geworden fast beliebige Proteine, Peptide oder deren Derivate in nahezu unbegrenzten Mengen herzustellen. Die Proteinreinigung erweist sich dabei häufig als der limitierende und nicht selten ineffiziente und damit letztlich kostenbestimmende Faktor.

Es wurden deshalb eine Reihe von Techniken zur Reinigung von Proteinen entwickelt. Für die Reinigung von Proteinen aus Zellüberständen, Zellrohextrakten oder Zellen werden üblicherweise Techniken wie Salzpräzipitation oder Präzipitation mit organischen Lösungsmitteln, Ultrafiltration, Dialyse, Gelelektrophorese, Isoelektrische Fokusierung, Chromatofokusierung, Ionenaustauscher- oder Gelchromatographie, Hydrophobe Chromatographie, Immunopräzipitation oder IMAC (= Immobilized Metal Affinity Chromatographie) verwendet. Es können einzelne Methoden zur Reinigung verwendet werden, in der Regel ist aber eine Kombination verschiedener Techniken erforderlich. Eine Übersicht über übliche Reinigungsmethoden ist beispielsweise den Lehrbüchern Protein Purification Process Engineering (Ed. R-G- Harrison, 1994, Marcel Dekker, Inc., New York, page 209 - 316, ISBN 0-8247-9009-X), Protein Purification (Ed. R.K. Scopes, 1994, Springer Verlag New York, chapter 4 - 7, ISBN 0-387-94072-3) und Methods for Protein Analysis (Ed. R.A. Copeland, 1994 Chapman & Hall, page 59 - 112, ISBN 0-412-03741-6) zu entnehmen. Für die Proteinreinigung ist es wichtig, daß die Reinigung so schonend wie möglich, so selektiv wie möglich und möglichst rasch erfolgt. Dabei sollen die Proteinverluste so gering wie irgend möglich gehalten werden. Viele der Proteinreinigungsmethoden sind nicht ausreichend selektiv, für nur geringe Proteinmengen geeignet und/oder sehr teuer.

Die von Porath et al. (Nature, Vol.256, 1975: 598 - 599) beschriebene Methode zur Proteinreinigung mit Hilfe der I_{MAC} (= Immobilized Metal Affinity Chromatographie) ist ein guter Kompromiss zwischen den genannten Anforderungen an eine optimale Reinigung. Diese Methode hat jedoch noch einige Nachteile. So binden beispielsweise nicht alle Metallionen gleich gut an das Trägermaterial, so daß die Ionen zum Teil ausgewaschen werden und so das Wertprodukt verunreinigen. Viele Proteine binden gar nicht an das Chromatographiematerial und lassen sich so nicht reinigen oder binden zu schwach, so daß sie bei den erforderlichen Waschschritten des Säulenmaterials schon eluiert werden. Dies führt zu unerwünschten Produktverlusten. Da die Selektivität in der Regel für eine "Ein-Schritt-Reinigung" nicht ausreichend ist, im Gegensatz zur Reinigung über biospezifische Affinitätsreinigungsmethoden wie beispielsweise eine Reinigung über Antikörper, sind weitere Reinigunsschritte erforderlich,

Um eine breitere Palette an Proteinen mit dieser Methode reinigen zu können, wurde verschieden sogenannte Tags wie Polyhistidin, His-Trp, His-Tyr oder (His-Asp)ₙ entwickelt (siehe Sporeno et al., J. Biol. Chem. 269 (15), 1994: 10991 - 10995, Le Grice et al., Eur. J. Biochem., 187 (2), 1990: 307 - 314, Reece et al., Gene, 126 (1), 1993: 105 - 107, De Vos et al., Nucl. Acid. Res., 22 (7), 1994: 1161 - 1166, Feng et al., J. Biol. Chem. 269 (3), 1994: 2342 - 2348, Hochuli et al., Biotechnology, 1988 : 1321 - 1325, Patwardhan et al., J. Chromatography A, 787, 1997: 91 - 100, Hutchens et al., J. Chromatography, 604, 1992: 133 - 141). Diese Tags werden mit den zu reinigenden Protein mit Hilfe der Molekularbiologie auf Nukleinsäureebene verbunden. Durch diese Tags konnte die Proteinreinigung in einigen Bereichen weiter verbessert werden. Jedoch auch diese Methode hat nach wie vor einige Nachteile. Nach wie vor läßt sich nicht sicher Vorhersagen, ob ein Protein über diese Methode aufgereinigt werden kann (siehe Immobilized Metal Ion Affinity Chromatography, L. Kagedal, page 227 - 251 in Protein Purification, Eds. J.C. Janson, L. Rydén, 1989, VCH Publishers, Inc., New York, ISBN 0-89573-122-3), das heißt auch diese Methode ist nicht auf jedes Protein anwendbar. Auch hier können Metallionen ausgewaschen werden oder Proteine so schwach binden, daß sie während der Waschschritte zum Teil verloren gehen. Auch die Selektivität läßt noch zu wünschen übrig. Außerdem ist die Beladungskapazität des Säulenmaterials mit den zu reinigenden Proteinen teilweise noch zu gering, so daß für eine Reinigung viel Säulenmaterial verwendet werden muß. Auch die Proteinausbeute ist noch nicht ausreichend. Dies führt zu unnötigen Kosten.

Von Volz et al. wurde die Reinigung zweier ATPasen von Helicobacter pylori ohne die Verwendung einer zusätzlichen His-Tag-Sequenz beschrieben. Diese ATPasen enthalten natürliche Metallbindungsstellen, die eine Reinigung über IMAC ermöglichen. Eigene Arbeiten zeigten, daß diese Bindungsstellen für eine effiziente Aufreinigung beliebiger Proteine eine zu geringe Bindungsaffinität aufweisen.

Es bestand daher die Aufgabe weitere Tags für die Proteinreinigung mittels IMAC zur Verfügung zu stellen, die die oben genannten Nachteile nicht besitzen und dadurch beispielsweise eine breitere Anwendung der Tags und/oder eine höhere Beladungsdichte der des Säulenmaterials ermöglichen. Die eine höhere Selektivität zeigen und so die Reinigung vereinfachen.

Die Aufgabe wurde durch die erfindungsgemäßen Peptidfragmente mit der allgemeinen Sequenz
His-X¹-His-X²-X³-X⁴-Cys-X⁵-X⁶-Cys gelöst,
wobei die Variablen X¹ bis X⁶ in der Sequenz die folgende Bedeutung haben:
- X¹=: eine Aminosäure ausgewählt aus der Gruppe Ala, Val, Phe, Ser, Met, Trp, Tyr, Asn, Asp oder Lys;
- X² =: eine Aminosäure ausgewählt aus der Gruppe Val, Ile, Phe, Pro, Trp, Tyr, Gln, Glu oder Arg;
- X³ =: eine Aminosäure ausgewählt aus der Gruppe Gly, Ile, Thr, Met, Trp, Tyr, Asn, Gln, Asp, Glu, Lys, Arg oder His;
- X⁴ =: eine Aminosäure ausgewählt aus der Gruppe Val, Phe, Pro, Cys, Met, Trp, Asn, Glu, Arg oder His;
- X⁵ =: eine Aminosäure ausgewählt aus der Gruppe Gly, Ser, Cys, Met, Trp, Asn, Glu, Lys oder Arg;
- X⁶=: eine Aminosäure ausgewählt aus der Gruppe Phe, Pro, Ser, Cys, Trp, Tyr oder Gln:

Vorteilhafterweise bedeutet in der Sequenz mindestens eine der Variablen X¹ bis X⁶ außerdem unabhängig voneinander Lys oder Arg. Weitere vorteilhafte in den Variablen X¹ bis X⁶ enthaltene Aminosäuren sind Glu, Lys, Arg, Tyr, Cys, Lys, His, Asp oder Met. Bevorzugt sind die Aminosäuren Cys, Glu, Lys, Tyr oder Arg enthalten, besonders bevorzugt Cys. Diese Aminosäuren tragen zu einer vorteilhaften Bindung der Peptidfragmente an die immobilisierten Metallionen bei. Außerdem sind vorteilhafterweise nicht mehr als vier bevorzugt drei Histidinreste in Folge in der Sequenz enthalten.

Weiter haben die Variablen X¹ bis X⁶ in der Sequenz unabhängig voneinander folgende vorteilhafte bevorzugte Bedeutung:
- X¹ =: eine Aminosäure ausgewählt aus der Gruppe Phe, Ser, Asn, Asp oder Lys, ganz besonders bevorzugt Asn;
- X³=: eine Aminosäure ausgewählt aus der Gruppe Val, Ile, Phe, Pro, Gln, Glu oder Arg, ganz besonders bevorzugt Gln, Glu oder Arg;
- X³ =: eine Aminosäure ausgewählt aus der Gruppe Gly, Ile, Thr, Met, Trp, Tyr, Asn, Asp, Glu, Arg oder His, ganz besonders bevorzugt Gly, Thr oder Tyr;
- X¹ =: eine Aminosäure ausgewählt aus der Gruppe Val, Phe, Cys, Met, Trp, Asn, Arg oder His, ganz besonders bevorzugt Asn oder Arg;
- X⁵ =: eine Aminosäure ausgewählt aus der Gruppe Gly, Ser, Cys, Met, Asn, Glu, Lys oder Arg, ganz besonders bevorzugt Gly oder Lys;
- X⁶ =: eine Aminosäure ausgewählt aus der Gruppe Phe, Ser, Cys, oder Tyr, ganz besonders bevorzugt Cys.

Die Variablen X¹ bis X⁶ in der Sequenz His-X¹-His-X²-X³-X⁴-Cys-X⁵-X⁶-Cys können unabhängig voneinander die verschiedenen bevorzugten Bedeutungen haben.

Besonders bevorzugte Peptidfragmente sind Fragmente mit den Sequenzen
His-Gln-His-Glu-Gly-Arg-Cys-Lys-Glu-Cys
His-Asn-His-Arg-Tyr-Gly-Cys-Gly-Cys-Cys
His-Arg-His-Gly-Thr-Asn-Cys-Leu-Lys-Cys
His-Ile-His-Gln-Ser-Asn-Cys-Gln-Val-Cys.

Die genannten Sequenzen entsprechen jeweils den Sequenzen SEQ ID NO: 2, SEQ ID NO: 3, SEQ ID NO: 4 und SEQ ID NO: 5.

Die oben genannte Proteinfragmentsequenz wird durch die erfindungsgemäßen Nukleinsäurefragmente kodiert. Hierbei ist der degenerierte genetische Code zu berücksichtigen. Die erfindungsgemäßen Nukleinsäurefragmente können prinzipiell in beliebigen Nukleinsäuren enthalten sein. Vorteilhafterweise werden die Nukleinsäurefragemente in Vektoren so inseriert, daß die Herstellung von zusammengesetzten Nukleinsäuresequenzen (= Genkonstrukte), die für die erfindungsgemäßen Fusionsproteine kodieren, ermöglicht wird. Diese Genkonstrukte können zur Expression vorteilhaft in einen geeigneten Wirtsorganismus verbracht werden, der eine optimale Expression des Fusionsproteins ermöglicht. Geeignete Vektoren sind dem Fachmann wohl bekannt und können beispielsweise aus dem Buch Cloning Vectors (Eds. Pouwels P. H, et al. Elsevier, Amsterdam-New York-Oxford, 1985, ISBN 0 444 904018) entnommen werden. Unter Vektoren sind außer Plasmiden auch alle anderen dem Fachmann bekannten Vektoren wie beispielsweise Phagen, Viren, Transposons, IS-Elemente, Plasmide, Cosmide, lineare oder zirkuläre DNA zu verstehen. Diese Vektoren können sich autonom im Wirtsorganismus replizieren oder chromosomal repliziert werden.

Unter den erfindungsgemäßen Nukleineäuresequenzen sind Sequenzen zu verstehen, die mit einem oder mehreren Regulationssignalen vorteilhafterweise zur Erhöhung der Genexpression funktionell verknüpft wurden. Diese Sequenzen können 3' und/oder 5' Terminale regulatorische Sequenzen zur Steigerung der Expression sein, die je nach ausgewähltem Wirtorganismus und Gen für eine optimale Expression ausgewählt werden. Beispielsweise handelt es sich bei diesen regulatorischen Sequenzen um Sequenzen an die Induktoren oder Repressoren binden und so die Expression der Nukleinsäuren regulieren. Das Genkonstrukt kann außerdem vorteilhafterweise auch eine oder mehrere sogenannte "enhancer Sequenzen" funktionell verknüpft mit dem Promotor enthalten, die eine erhöhte Expression der Nucleinsäuresequenz ermöglichen. Dies kann beispielsweise über eine verbesserte Wechselwirkung zwischen RNA-Polymerase und DNA erfolgen. Auch am 3'-Ende der DNA-Sequenzen können zusätzliche vorteilhafte Sequenzen inseriert werden wie weitere regulatorische Elemente oder Terminatoren. Die erfindungsgemäßen Nukleinsäurefragmente werden vorteilhaft im Vektor so inseriert, daß sie die N-terminale Region des Fusionsproteins bilden. Sie können jedoch auch am C-Terminus lokalisiert sein oder aber innerhalb des Proteins liegen, wobei jedoch die Funktion des Proteins nicht beeinflußt sein darf und ein Ausschneiden aus dem Fusionsprotein nicht mehr möglich ist.

Die regulatorischen Sequenzen sollen die gezielte Expression der Gene und der Proteinexpression ermöglichen. Dies kann beispielsweise je nach Wirtsorganismus bedeuten, daß das Gen erst nach Induktion exprimiert oder überexprimiert wird, oder daß es sofort exprimiert und/oder überexprimiert wird.

Vorteilhafte Regulationssequenzen sind beispielsweise in Promotoren wie cos-, tac-, trp-, tet-, trp-tet-, Ipp-, lac-, lpplac-, lacI^{a-,} T7-, T5-, T3-, gal-, trc-, ara-, SP6-, λ-P_{R}- oder im λ-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFα, AC, P-60, CYC1, GAPDH, TEF, rp28, ADH oder in den Pflanzenpromotoren CaMV/35S, SSU, OCS, lib4, usp, STLS1, B33, nos oder im Ubiquitin- oder Phaseolin-Promotor enthalten. In diesem Zusammenhang sind auch die Promotoren der Pyruvatdecarboxylase und der Methanoloxidase aus beispielsweise Hansenula vorteilhaft. Es können auch künstliche Promotoren für die Regulation verwendet werden.

Die regulatorischen Sequenzen bzw. Faktoren können dabei vorzugsweise die Genexpression des eingeführten Gens positiv beeinflussen und dadurch erhöhen. So kann eine Verstärkung der regulatorischen Elemente vorteilhafterweise auf der Transkriptionsebene erfolgen, indem starke Transkriptionssignale wie die genannten Promotoren und/oder "Enhancer" verwendet werden. Daneben ist aber auch eine Verstärkung der Translation möglich, indem beispielsweise die Stabilität der mRNA verbessert wird.

Vorteilhafterweise enthalten die erfindungsgemäßen Nukleinsäuresequenzen auch Signale, die eine Sekretion der Proteine ins Medium oder in Zellkompartimente ermöglichen. Als Beispiel für derartige Sequenzen seien die typischen Signalsequenzen wie beispielsweise die Signalsequenz von ompA (E. coli-Membranprotein) genannt. Daneben können weitere vorteilhafte Sequenzen wie die Sequenz des α-Faktor enthalten sein oder sog. YACS (= Yeast artifial chromosomes) verwendet werden.

Die erfindungsgemäßen Genkonstrukte (= Nukleinsäuresequenzen) enthalten vorteilhafterweise außerdem noch Sequenzen, die eine Abspaltung der erfindungsgemäßen Proteinfragmente mit der oben genannten Sequenz vom N- oder C-Terminus des Fusionsproteins bevorzugt vom N-Terminus ermöglichen. Diese Sequenzen kodieren beispielsweise für Schnittstellen verschiedenster Proteasen wie beispielsweise für Faktor Xa, Enterokinase, humanen Renin, Carboxypeptidase A, Thrombin, Trypsin, Dipeptidylpepdidasen, Papain, Plasmin, Pepsin oder sonstigen Proteasen. Bevorzugt werden Schnittstellen für Faktor Xa, humanen Renin, Dipeptidiylpeptidasen, Carboxypeptidase A oder Enterokinase, da diese Enzyme eine hohe Spezifität besitzen und so ein unerwünschter Verdau des zu reinigenden Proteins vermieden werden kann. Werden andere Proteasen verwandt, so ist darauf zu achten, daß keine Schnittstellen innerhalb des zu reinigenden Proteins sind. Das Proteinfragment kann auch über eine Bromcyanspaltung [z.B 2-(2-Nitrophenylsulfenyl)-3-brom-3' -methylindolinium, Hydoxylamin etc.] in Gegenwart von Ameisensäure entfernt werden. Hierbei ist jedoch in der Regel eine Rückfaltung des Proteins erforderlich, was dazu führt, daß diese Methode weniger bevorzugt ist. Auch eine Abspaltung des Proteinfragments über einen Exoproteaseverdau (kinetisch kontrolliert) ist möglich. Hierbei entstehen jedoch in der Regel Produktgemische. Bevorzugt werden Schnittstellen verwendet, die eine Entfernung der Proteinfragmente ohne das Zurücklassen von Proteinfragmentresten im zu reinigenden Protein ermöglichen. Können im Fusionsprotein die erfindungsgemäßen Proteinfragmente ohne Funktionsverlust und ohne sonstige Nachteile toleriert werden, so kann auf eine spezielle Stelle zur Abtrennung des Proteinfragments verzichtet werden.

Als Vektoren sind prinzipiell alle Vektoren geeignet, die eine Expression in pro- oder eukaryontischen Zellen ermöglichen. Dabei können Vektoren verwendet werden, die nur in einer Gattung replizieren oder solche, die in mehreren Gattungen replizieren (= sog. shuttle-vectoren). Vorteilhafte Vektoren sind beispielsweise Plasmide wie die E. coli-Plasmide pEGFP, pLG338, pACYCl84, pBR322, pUC18, pUC19, pKC30, pRep4, pHS1, pHS2, pPLc236, pMBL24, pLG200, pUR290, pIN-III¹¹³-B1, λgt11 oder pBdCI, bevorzugt pEGFP, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2∝M, pAG-1, YEp6, YEp13 oder pEMBLYe23 oder in Pflanzen pLGV23, pGHlac⁺, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem oben genannten Buch Cloning Vectors (Eds. Pouwels P. H. et al. Elsevier, Amsterdam-New York-Oxford, 1985 , ISBN 0 444 904018) entnommen werden.

In einer weiteren Ausgestaltungsform des Vektors kann die erfindungsgemäße Nukleinsäuresequenz auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus der Nukleinsäure, d.h. dem Nukleinsäurefragment und dem Gen für das Protein (= Fusionsproteingen) sowie gegebenenfalls weiteren regulatorischen Sequenzen bestehen.

Als Wirtsorganismen für das erfindungsgemäße Genkonstrukt kommen prinzipiell alle prokaryontischen oder eukaryontischen Organismen in Frage. Vorteilhafterweise werden als Wirtsorganismen Mikroorganismen wie gram-positive oder gram-negative Bakterien, Archaebakterien, Pilze, Hefen, tierische oder pflanzliche Zellen wie Drosophila speziell D. melanogaster, Maus, Zebrafisch oder Tabak verwendet. Bevorzugt werden gram-positive oder gram-negative Bakterien, Pilze oder Hefen, besonders bevorzugt die Gattungen Escherichia, Bacillus, Streptomyces, Aspergillus oder Saccharomyces, ganz besonders bevorzugt E. coli verwendet.

Besonders bevorzugt sind folgende Kombinationen aus Vektor und Wirtsorganismus wie Escherichia coli und seinen Plasmiden und Phagen und den dazugehörenden Promotoren sowie Bacillus und seine Plasmide und Promotoren, Streptomyces und seine Plasmide und Promotoren, Aspergillus und seine Plasmide und Promotoren oder Saccharomyces und seine Plasmide und Promotoren zu verstehen.

Die erfindungsgemäßen Fusionsproteine lassen sich wie oben beschrieben in einem Verfahren herstellen, wobei die erfindungsgemäßen Nukleinsäurefragmente, die für die Proteinfragmente mit der oben genannten Sequenz kodieren, mit einem Gen, das für die zu reinigenden Proteine kodiert, und gegebenenfalls weiteren vorteilhaften Sequenzen wie Promotor-und/oder Enhancersequenzen, Schnittstellen für Proteasen etc. fusioniert werden. Dazu wird falls erforderlich eine geeignete Restriktionsenzymschnittstelle zwischen dem Nukleinsäurefragment und dem Gen des zu reinigenden Proteins eingeführt und dieses Konstrukt über geeignete Schnittstellen in einen Vektor inseriert. Derartige Methoden sind dem Fachmann bekannt und können beispielsweise aus den Lehrbüchern von Sambrook, J. et al. (1989) Molecular cloning: A laboratory manual, Cold Spring Harbor Laboratory Press, von F.M. Ausubel et al. (1994) Current protocols in molecular biology, John Wiley and Sons oder D.M. Glover et al., DNA Cloning Vol.1, (1995), IRL Press (ISBN 019-963476-9) entnommen werden. Weitere vorteilhafte Vektoren sind die Pichia pastoris Vektoren pPic und pGap. Diese Hefe ist auch ein geeigneter Wirtsorganismus für die Proteinexpression.

Die erfindungsgemäßen Proteinfragmente eignen sich zur Herstellung von Fusionsproteinen, die mit Hilfe der Proteinfragmente leicht, kostengünstig und effizient aufgereinigt werden können. Die erfindungsgemäßen Proteinfragmente und Fusionsproteine können so vorteilhafterweise sehr selektiv in guten Ausbeuten, mit hoher Reinheit gereinigt werden. Die erfindungsgemäßen Proteinfragmente und damit die aus ihnen hergestellten Fusionsproteine zeichnen sich vorteilhaft durch eine im Vergleich zur Helicobacter pilori ATPase-439 Sequenz um mindestens 1,5 fach stärkere Bindung an die immobilisierten Metallionen aus.

Zur Herstellung der Fusionsproteine sind prinzipiell alle Proteine geeignet. Bevorzugt werden Proteine verwendet, die eine biologische Wirkung in Mensch, Tier oder Pflanze zeigen oder die für organische Synthese interessant sind. Hierbei handelt es sich beispielsweise um Proteine wie Enzyme, Hormone, Speicher- oder Bindungs- oder Transportproteine. Beispielhaft seien Proteine wie Hydrolasen wie Lipasen, Esterasen, Amidasen, Nitrilasen, Proteasen, Mediatoren wie Cytokine z.B. Lymphokine wie MIF, MAF, TNF, Interleukine wie Interleukin 1, Interferone wie γ-Interferon, tPA, Hormone wie Proteohormone, Glykohormone, Oligo- oder Polypetidhormone wie Vassopressin, Endorphine, Endostatin, Angiostatin, Wachstumsfaktoren Erythropoietin, Transkriptionsfaktoren, Integrine wie GPIIb/IIIa oder αᵥβIII, Rezeptoren wie die verschiedenen Glutamatrezeptoren, Angiogenesefaktoren wie Angiotensin genannt.

Das erfindungsgemäße verfahren zur Reinigung von Fusionsproteinen ermöglicht beispielsweise die Reinigung von Proteinen aus natürlichen Quellen wie Pflanzen- oder Tierextrakten, Pflanzen- oder Tierzell-Lysaten, aus Kulturmedien, Fermentationsbrühen oder aus Synthesebrühen um nur einige beispielhaft zu nennen.

Das erfindungsgemäße Verfahren umfaßt folgende Reaktionsschritte:
a) Die Flüssigkeit(en), die das Fusionsprotein enthalten, werden so mit immobilisierten Metallionen in Kontakt gebracht, daß sich zwischen den Metallionen und dem Fusionsprotein eine Affinitätsbindung ausbilden kann,
b) Entfernen nicht gebundener in der Flüssigkeit enthaltener Substanzen,
c) Eluieren des gebundenen Fusionsproteins, in dem die Affinitätsbindung durch Änderung des Flüssigkeitsmileus aufgehoben wird und
d) Sammlung des gereinigten Fusionsprotein.

Vorteilhaft wird das Fusionsprotein in einem geeigneten Wirtsorganismus (siehe oben) vor der Reinigung exprimiert um die Ausbeute an Fusionsprotein zu erhöhen. Der Wirtsorganismus wird in einem geeignetem synthetischen oder komplexen Medium, das eine Kohlenstoffquelle, eine Stickstoffquelle und gegebenenfalls anorganische Salze, Vitamine und Spurenelemente enthält, bei geeigneter Temperatur und Belüftung angezogen.

Je nach dem ob das Fusionsprotein aus den Zellen exkretiert wird oder nicht, werden die Zellen zunächst aufgeschlossen und die Zellen oder Zelltrümmer vorteilhafterweise abgetrennt. Für den Zellaufschluß werden dem Fachmann bekannte Methoden verwendet wie Ultraschall, French press, Enzymaufschluß, osmotischer Schock und andere mehr. Die Abtrennung der Zellen oder Zelltrümmer kann beispielsweise über Zentrifugation oder Filtration erfolgen. Eine Abtrennung der Zellen oder Zelltrümmer ist jedoch nicht zwingend erforderlich.

Die die Fusionsproteine enthaltende Flüssigkeit wird anschließend mit den immobilisierten Metallionen in Kontakt gebracht, so daß sich eine Affinitätsbindung zwischen dem Fusionsprotein und den Metallionen ausbilden kann. Die Bindung erfolgt bei pH-Werten größer 7, beispielsweise vorteilhaft bei pH 7,0 bis 9,0 bevorzugt zwischen pH 7,5 bis 8,0. Vorteilhafte Puffer sind einzelne Puffer oder Puffergemische wie beispielsweise 50 bis 1000 mM Puffer wie 50 mM Tris/HCl pH 8,0 + 150 mM NaCl, 100 mM NaAcetat pH 7,7 + 500 mM NaCl, 20 mM NaPhosphat pH 7,7 + 500 mM NaCl oder 50 mM Tris/HCl pH8,0 + 150 mM NH₄Cl. Diese Puffer ermöglichen ein Auftragen der Fusionsproteine auf die immobilisierten Metallionen. Im einfachsten besonders vorteilhaften Fall werden die Fusionsproteine direkt im zum Aufschluß verwendeten Puffer oder im Inkubationsmedium mit den immobilisierten Metallionen in Kontakt gebracht. Vorteilhafterweise werden die Flüssigkeiten und die immobilisierten Metallionen in einer üblichen Chromatographiesäule miteinander in Kontakt gebracht. Dies erleichtert das Entfernen nicht gebundener Substanzen beispielsweise Proteine durch Waschen der Säule mit einem geeigneten Puffer. Geeignete Puffer sind Puffer, die die Bindung der erfindungsgemäßen Proteinfragmente oder der Fusionsproteine an die Metallionen nicht stören und Verunreinigungen entfernen können. Solche Puffer haben vorzugsweise einen pH-Wert größer pH7, vorteilhaft einen pH-Wert zwischen pH 7,0 bis 9,0, bevorzugt zwischen pH 7,5 bis 8,0. Auch batch-Ansätze, bei denen die immobilisierten Metallionen in einem Gefäß vorgelegt werden und die Flüssigkeiten anschließend zugesetzt werden oder umgekehrt, können so gereinigt werden. Für diese batch-Ansätze können die genannten Puffer verwendet werden. Zwischen den einzelnen Waschschritten werden die Ansätze vorteilhafterweise zentrifugiert oder filtriert.

Für die Immobilisierung der Metallionen eignen sich prinzipiell alle üblichen Trägermaterialen, die sich leicht derivatisieren lassen, keine oder nur geringe unspezifische Adsorption aufweisen, gute physikalische, mechanische und chemische Stabilität aufweisen und eine hohe äußere und innere Oberfläche aufweisen. Geeignete Materialien sind beispielsweise käuflich erwerbbar von Pharmacia LKB, Schweden (Sepharose^{™}6B oder Superose^{™}), Pierce, USA (immobilisierte Iminodiessigsäure I oder II, immobilisiertes Tris(carboxymethyl)ethylendiamin), Sigma, USA (immobilisierte Imminodiessigsäureagarose), Boehringer Mannheim, Deutschland (zinkchelatagarose) oder Toyo Soda, Japan (TSKgel Chelate-5PW). In EP-B-0 253 303 werden weitere geeignete Materialien beschrieben. Weitere geeignete vorteilhafte Materialien sind Materialien wie Ni-beschichtete Mikrotiterplatten (Nickel-chelate coated Flashplate^{®}, Nen life science products) oder Magnetpartikel oder speziell mit Metallionen behandelte und bindende Membranen.

Die verschiedenen Metallionen werden in geeigneten Materialien vorteilhaft über Gruppen wie IDA (= Imminodiessigsäure), NTA (= Nitrilotriessigsäure) oder TED (= Tris(carboxymethyl) ethylendiamin gebunden. Geeignete Metallionen sind Co, Cu, Fe, Ca, Mg, Ni, Al, Cd, Hg oder Zn, bevorzugt werden Fe, Ni oder Cu, besonders bevorzugt Ni oder Cu, ganz besonders bevorzugt Ni. Die Beladung der Materialien mit Metallionen erfolgt vorteilhaft mit 0,1 bis 0,4 M Lösungen der Metallsalze in wäßriger, ungepufferter Lösung.

Nach dem Waschen wird das Fusionsprotein mit einem geeigneten Puffer eluiert. Dieser Puffer hebt die Affinitätsbindung zwischen dem Fusionsprotein und den immobilisierten Metallionen auf. Die Fusionsproteine können über einen pH-Gradienten (niedrige pH-Werte < pH 7,0 wirken eluierend), kompetitive Liganden wie Imidazol, organische Lösungsmittel wie Aceton oder Ethanol, chelatisierende Agentien wie EDTA oder NTA und/oder Detergentien wie Tween 80 eluiert werden. Bevorzugt wird eine Elution über kompetitive Liganden wie Imidazol und/oder Detergentien. Imidazol wird in einem Bereich von 0,05 bis 0,7 M, bevorzugt von 0,1 bis 0,5 M zur Elution verwendet. Die kompetitiven Liganden und/oder Detergentien werden vorteilhaft in einem Puffer eingesetzt, aber auch die Verwendung in Wasser ist möglich. Vorteilhafte Puffer sind Puffer, die den Puffern entsprechen, die für das Auftragen auf die immobilisierten Metallionen verwendet wurden. Dies hat den Vorteil, daß keine unerwünschten Wechselwirkungen zwischen dem Säulenmaterial, den gebundenen Proteinen und dem Puffer auftreten. Vorteilhafte Puffer haben vorzugsweise einen pH-Wert größer pH7, vorteilhaft einen pH-Wert zwischen pH 7,0 bis 9,0, bevorzugt zwischen pH 7,5 bis 8,0. Bevorzugt werden diese Puffer über einen steigenden Gradienten aufgebracht. Im Falle, daß mit einem pH-Gradient eluiert wird, können Puffer mit einem pH-Wert kleiner pH 7,0 und/oder Säuren verwendet werden. Das eluierte Fusionsprotein wird gesammelt und kann sofort verwendet werden, oder aber falls erforderlich und gewünscht weiter behandelt werden. Geeignete Auftrags- und Elutionspuffer sind beispielsweise dem Lehrbuch Protein Purification (Eds. J.C. Janson, L. Rydén, VCH Publisher Inc., 1989, Seite 227 bis 251) zu entnehmen.

Das erfindungsgemäße Proteinfragment kann mit den oben beschriebenen Methoden wie Bromcyan- oder Proteasespaltung entfernt werden. Hierbei können Reste des Proteinfragments im Molekül enthalten bleiben oder aber total aus dem zu reinigenden Protein abgespalten werden. Vorteilhaft wird das Proteinfragment rückstandslos aus dem Protein entfernt.

Vorteilhaft geeignete Proteinfragemente für die Herstellung von Fusionsproteinen lassen sich durch folgendes erfindungsgemäße Verfahren screenen. Die Erfindung betrifft ein Verfahren zur Herstellung von Proteinfragmenten, die in der Lage sind mit immobilisierten Metallionen eine reversible Affinitätsbindung einzugehen, dadurch gekennzeichnet, daß man folgende Schritte durchführt:
a) Herstellung einer Nukleinsäurebibliothek ausgehend von einer beliebigen Nukleinsäuresequenz, die für ein Proteinfragment der Sequenz
   His-X¹-His-X²-X³-X⁴-Cys-X⁵-X⁶-Cys,
   kodiert, wobei die Histidin- und Cysteinreste der Sequenz in der Nukleinsäurebibliothek konserviert werden,
b) Fusion der Nukleinsäuren der Bibliothek an ein Reportergen, das die Detektion des durch die erhaltene Nukleinsäure kodierten Fusionsproteins über seine Bindung an die immobilisierten Metallionen ermöglicht und
c) Auswahl der Nukleinsäuresequenzen, die eine gegenüber der in der natürlichen Helicobacter pilori ATPase-439 Sequenz um mindestens 1,5 fach stärkere reversible Bindung an die immobilisierten Metallionen aufweisen.

Die Nukleinsäurebibliothek ausgehend von der oben genannten Sequenz läßt sich über dem Fachmann bekannte Methoden zur Mutagenese erstellen. Hierzu kann die Sequenz beispielsweise einer "site directed mutagenesis" unterzogen werden wie sie in D.M. Glover et al., DNA Cloning Vol.1, (1995), IRL Press (ISBN 019-963476-9), Kapitel 6, Seite 193 ff beschrieben wird.

Von Spee et al. (Nucleic Acids Research, Vol. 21, No. 3, 1993: 777 - 778) wird eine PCR-Methode unter Verwendung von dITP zur zufälligen Mutagenese beschrieben.

Die Verwendung einer "in vitro" Rekombinationstechnik für die molekulare Evolution wird von Stemmer (Proc. Natl. Acad. Sci. USA, Vol. 91, 1994: 10747 - 10751) beschrieben.

Von Moore et al. (Nature Biotechnology Vol. 14, 1996: 458 - 467) wird die Kombination der PCR- und Rekombinationsmethode beschrieben.

Die Verwendung einer "in vitro" Rekombinationstechnik für die molekulare Evolution wird von Stemmer (Proc. Natl. Acad. Sci. USA, Vol. 91, 1994: 10747 - 10751) beschrieben. Auch die Kombination der beiden Methoden kann verwendet werden.

Die Verwendung von Mutationsstämmen mit Defekten im DNA-Reparatursystem wird von Bornscheuer et al. (Strategies, 11, 1998: 16 - 17) beschrieben. Von Rellos et al. wird eine PCR-Methode unter Verwendung von nichtäquimolaren Nucleotidmengen (Protein Expression and Purification, 5, 1994: 270 - 277) beschrieben.

Vorteilhaft läßt sich die Nukleinsäurebibliothek über eine PCR-Technik mit Hilfe zweier komplementärer, degenerierter Oligonukleotide (= sog. wobble-primer), wie in den Beispielen beschrieben, erzeugen. Wichtig ist, daß die in der Sequenz enthaltenen Histidin- und Cysteinreste konserviert werden.

Zum Screening auf Proteinfragmente mit einer verbesserten Metallbindungsaffinität wird das erfindungsgemäße Nukleinsäurefragment, das für das Proteinfragment kodiert, an ein Reportergen fusioniert. Vorteilhafte Reportergene ermöglichen eine leichte Detektion der Bindung an die immobilisierten Metallionen über beispielsweise eine Bindung von mit einem Fluoreszenzfarbstoff markierten Antikörpern, die gegen das Reportergen gerichtet sind, oder über selbst fluoreszierende Proteine wie das vorteilhafte bevorzugte egf-Protein von E. coli (= green fluorescent protein, siehe Prasher et al., Gene 111 (2), 1992: 229 - 233) oder das bevorzugte Biolumineszenzprotein von Aequoria victoria oder über Licht generierende Proteine wie das Luzeferin/Luzeferase-System. Besonders bevorzugt wird ein gfp-Proteinmutante (= egfp = enhanced green fluorescence protein) mit einer 35fach höheren Fluoreszenzaktivität, die durch zwei Punktmutationen an Position 64 Austausch von Phe gegen Leu und Position 65 Austausch von Ser gegen Thr verursacht wird, verwendet. Diese Proteinmutante hat den Vorteil gegenüber dem Wildtypprotein, daß es löslich ist und keine sog. "Inclusion Bodies" bildet. Die Verwendung des egfp-Proteins ermöglicht die Lokalisierung und Quantifizierung der Proteinkonzentration in jeder Phase der Reinigung der Proteine ohne Eingriff in die Reinigung und ohne Verwendung von weiterer Kofaktoren oder Substrate (Poppenborg et al., J. Biotechnol., 58 (2), 1997, 77 - 88). Außerdem zeichnet sich das egfp-Protein durch eine hohe Stabilität gegenüber einem weiten pH-Bereich (pH 5,5 bis 12), Bleichung durch Photooxidation, oxidierende und schwach reduzierende Agentien wie 2% Mercaptoethanol aus. Das Protein zeigt eine Abnahme der Fluoreszenz oberhalb 37 °c. Ebenfalls geeignet als Reportergen sind das gfp-uv-(blue fluorescence) und das eyfp-(yellow fluorescence)Protein.

Die Auswahl der geeigneten Sequenzen erfolgt im Vergleich zur Bindungsaffinität an die immobilisierten Metallionen der folgenden natürlichen Helicobacter pilori ATPase-439 Sequenz His-Ile-His-Asn-Leu-Asp-Cys-Pro-Asp-Cys. Die erfindungsgemäßen Proteinfragmentsequenzen weisen eine um mindestens 1,5 fach stärkere reversible Bindung an die immobilisierten Metallionen auf, bevorzugt eine mindestens zweifach, besonders bevorzugt mindestens dreifach stärkere reversible Bindung auf. Vorteilhafte Sequenzen ermöglichen eine Proteinausbeute nach der Reinigung von mindestens 20%, bevorzugt mindestens 30%, besonders bevorzugt von mindestens 40%, ganz besonders bevorzugt von mindestens 50%.

Das erfindungsgemäße Verfahren zum Screening der Nukleinsäurebibliothek eignet sich vorteilhaft für eine Automatisierung. Mit diesem Verfahren läßt sich einfach ein große Zahl von Nukleinsäurefragmenten bzw. Proteinfragmenten auf ihre Metallionenbindungsaffinität in einem sogenannten "High-Throughput-Screening" testen.

Mit den erfindungsgemäßen Proteinfragmenten lassen sich Proteine leicht detektieren. Im erfindungsgemäßen Verfahren zur Detektion von Proteinen werden aus einer Proteinmischung einzelne Proteine, die ein Proteinfragment mit den erfindungsgemäßen oben genannten Proteinfragmenten enthalten, über Antikörper nachgewiesen, die gegen das Proteinfragment gerichtet sind. Der Nachweis dieser Fusionsproteine erfolgt vorteilhaft über mono- oder polyklonale Antikörper, die gegen das Proteinfragment (= Tag) gerichtet sind. Das Proteingemisch kann vorteilhaft vor dem Nachweis chromatographisch oder elektrophoretisch aufgetrennt werden und anschließend auf eine geeignete Membran (z.B. PVDF oder Nitrocellulose) mit üblichen Methoden (siehe Sambrook et al.) überführt (= geblottet) werden. Anschließend wird diese Membran mit dem gegen das Tag gerichteten Antikörper inkubiert. Vorteilhaft wird die Membran mehrmals gewaschen und danach die gebundenen Antikörper über eine spezifische Reaktion mit einem beispielsweise Enzymkonjugierten (z.B. alkalische Phosphatase, Peroxidase etc.) zweiten Antikörper der gegen die konstante Region des ersten gerichtet ist in einem sog. Western- oder Immuno-Blot nachgewiesen. Entsprechende Antikörper sind im Handel erhältlich. Im Falle der Verwendung von Magnetpartikel kann auf das Waschen verzichtet werden, die mit Antikörpern beschichteten Magnetpartikel können über das Fischen mit Magneten gereinigt werden.

Die erfindungsgemäßen Proteinfragmente haben gegenüber den üblichen His-Tags bei der Proteindetektion den Vorteil, daß sie eine stärkere antigene Wirkung besitzen und so leichter zur Herstellung von Antikörpern gegen das Tag geeignet sind.

Die Erfindung wird durch die folgenden Beispiele weiter veranschaulicht.

### Beispiele:

Für die Bindungstest an Metallchelatsäulen (= immobilisierte Metallionen) wurde die sog. "Chelating Sepharose Fast-Flow" von Pharmacia LKB, Uppsala, Schweden verwendet. Ampicillin, Imidazol, EDTA und alle weiteren Reagentien wurden von Fluka (Buchs, Schweiz) bezogen. Das verwendete DNA Gel-Extraktions-Kit, das Midi Plasmid-Kit und das Prepspin Plasmid Kit stammten von Qiagen (Hilden, Deutschland), die Restriktionsenzyme, die DNAmodifizierenden Enzyme, die T4-DNA-Ligase und die Taq-Polymerase stammten von MBI Fermentas (St. Leon-Rot, Deutschland). Der Taq Dye Cycle Sequencing Kit wurde von Applied Biosystems (Weiterstadt, Deutschland) bezogen.

Für die Klonierungsversuche wurde der E. coli Stamm DH5α (F⁻ endA1 hsdR17 [rk⁻, mk⁺] supE44 thi1 λgyrA96 relA1 Δ (argF laczya) U169) verwendet. Die Plasmide, die das Gen für das egf-Protein enthielten, wurden von Clonetech USA bezogen. Die E. coli Stämme wurden bei 37 °C in Luria-Bertani Medium (= LB) mit 100 ∝g/ml Ampicillin zur Selektion der Klone auf Transformation mit dem egfp-Vektor angezogen. Der Lysepuffer enthielt 50 mM NaPhosphatpuffer pH 8,0, 300 mM NaCl, 1 mg/ml Lysozym und 1 mM PMSF (= Phenylmethansulfonylflourid = spez. Trypsin- und Chymotrypsin-Inhibitor).

Die DNA-Methoden wie Ligationen, Restriktionen, PCR oder Transformationen etc. wurden wie bei Sambrook, J. et al. (1989) Molecular cloning: A laboratory manual, Cold Spring Harbor Laboratory Press oder F.M. Ausubel et al. (1994) Current protocols in molecular biology, John Wiley and Sons beschrieben durchgeführt. Für die Sequenzierung wurde die Fluoreszenzmarkierte Dideoxy-DNA-Sequenzierungsmethode verwendet. Die DNA-Sequenzierung wurde mit dem Taq Dye Deoxy^{™} Cycle Sequencing Kit (Applied Biosystems) und dem 373A DNA Sequencing System (Applied Biosystems) nach Angaben des Herstellers durchgeführt.

### Beispiel 1: Herstellung zufällig mutagenisierter N-terminaler Metallbindungsstellen, die an das egfp-Gen gebundenen wurden, und des his6-egfp

Für die PCR-Reaktion wurde das Plasmid egfp und die zwei folgenden komplementäre Oligonukleotide
5'-GCAATACCATGGGGCATNNNCATNNNNNNNNNTGTNNNNNNTGTGTGAGGAAGGGCGAG-3'
5'-CAGTTGGAATTCTAGAG-3'
verwendet. Im Falle des his6-egfp wurden die folgenden zwei komplementären Primer
5' -GCAATACCATGGGGCATCATCATCATC'ATCATGTGAGGAAGGGCGAG-3 '
5'-CAGTTGGAATTCTAGAG-3'
verwendet.

Die für die PCR-Reaktionen verwendeten Bedingungen waren wie folgt:

| | | |
|---|---|---|
| Ansatz: | 8 | µl dNTP Mix (200 µmol) |
| | 10 | µl 10 x ThermoPolPuffer (New England Biolabs) |
| | 1 | µl (100 pmol) Primer1 |
| | 1 | µl (100 pmol) Primer2 |
| | 1 | µl (100 ng) egfp Plasmid |
| | 79,5 | µl Wasser |
| | 1 | µl Deep Vent Polymerase (New England Biolabs) |

| | |
|---|---|
| PCR-Programm | 95°C 7min |
| | 95 °C 1min |
| | 56 °C 1min 30x |
| | 72 °C 3min |
| | 72 °C 7min |

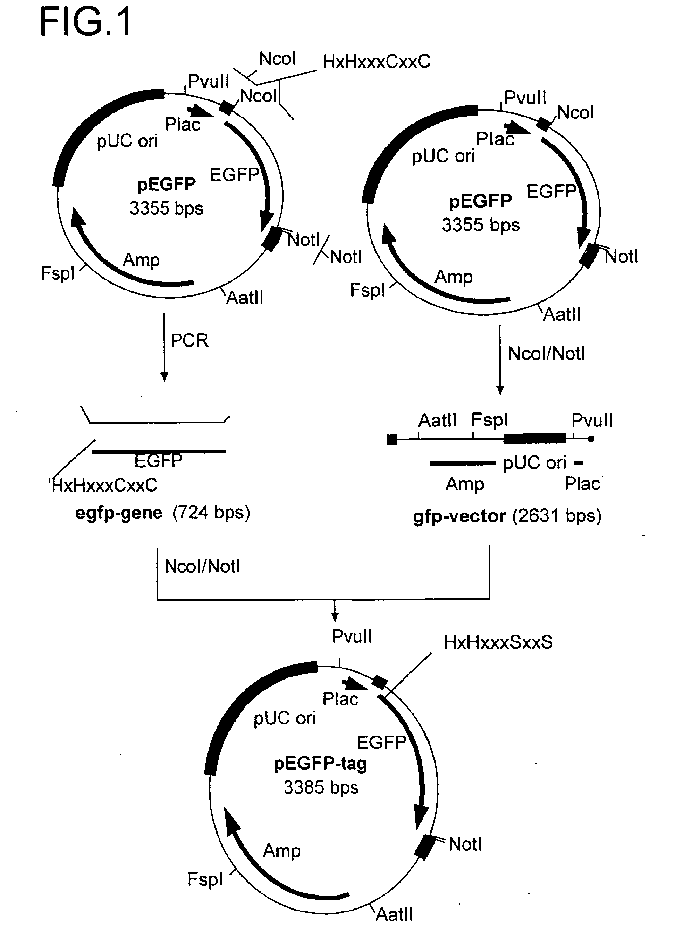

Die PCR-Produkte wurden jeweils mit NcoI und NotI verdaut und in den egfp-Vektor, der mit den gleichen Enzymen verdaut worden war, um Mutationen im Vektor auszuschließen legiert (siehe Figur 1). Die PCR-Vektorligationen wurden zur Transformation von E. coli verwendet. Die Transformanden wurden auf LB-Agar mit 100 µ g/ml Ampicillin plattiert und bei 37 °C inkubiert.

### Beispiel 2: Kultivierungsbedingungen und Herstellung der Zell-Lysate

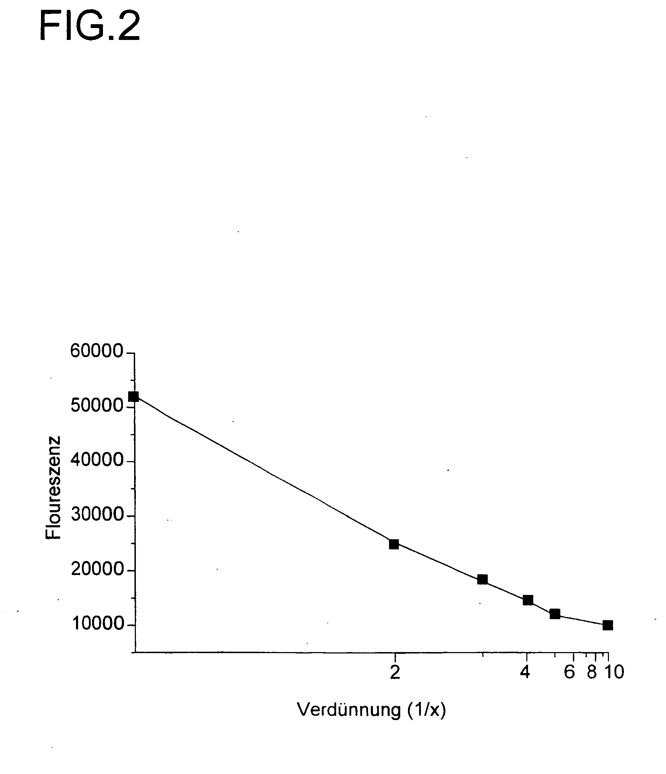

Transformierte Kolonien, die Fluoreszenz und einige die keine Fluoreszenz zeigten, wurden ausgewählt und in 50 ml LB-Medium, das 100 µg/ml Ampicillin enthielt, angezogen. Für das High-Throughput-Screening wurden Kolonien ausgewählt, die Fluoreszenz zeigten, und in sterilen Mikrotiterplatten, die 250 µl LB-Medium mit 100 µg/ml Ampicillin enthielten, inkubiert. Nach Inkubation wurden die Kulturen zentrifugiert. Die Pellets wurden in 2 ml Lysepuffer resuspendiert, 20 Minuten auf Eis inkubiert und anschließend mit Ultraschall aufgeschlossen (zweimal, 5 Minuten mit einem Branson Sonifier 250). Nach Zentrifugation (15 min, 4 C, 20000 x g) wurden die verschiedenen egfp-Mutanten im Überstand erhalten. Alle ausgewählten Klone wurden sequenziert (siehe Tabelle I und II). Klone, die keine Fluoreszenz zeigten, enthielten Stopcodons in der Sequenz, so daß keine funktionellen Proteine exprimiert wurden (siehe Tabelle I, A8, A13, M16a, Z4, Z11 und Z13). In allen Versuchen wurde zur Quantifizierung der gebundenen Proteine eine Fluoreszenzmessung durchgeführt, die in einem weiten Bereich mit der gfp-Konzentration korrelierte (siehe Figur 2).

### Beispiel 3: Low-Throughput-Screening mit Ni-NTA-Säulen von Quiagen

600 µl der lysierten Zellen wurden auf eine Ni-NTA-Säule aufgebracht, zweimal mit 600 µl einer Waschlösung (50 mM NaPhosphatpuffer, pH 8,0, 250 mM NaCl) gewaschen und anschließend mit einer 0,7 M Imidazollösung eluiert.

### Beispiel 4: High-Throughput-Screening mit Membranfilterplatten

Für das High-Throughput-Screening wurden Membranfilter-Mikrotiterplatten (MultiScreen 5 µm; Fa. Millipore, Molsheim, Deutschland) verwendet. In jedes "Well" der Membranfilterplatten wurden je dreimal 250 µl einer gerührten Chelatsepharosesuspension gegeben. Nach jeder Zugabe wurde die Sepharose abzentrifugiert (2 min, 23 °C, 350 rpm). Alle weiteren Schritte wurden in einem Beckmann Biomek 2000 Roboter durchgeführt. Die Minisäulen in den Wells wurden zweimal mit 250 µl Wasser gewaschen. Anschließend wurde die Sepharose mit mit 250 µl einer Metallsalzlösung beladen und dreimal mit 200 µl Puffer (50 mM NaPhosphat, pH 8,0, 250 mM NaCl) äquilibriert. Für die verschiedenen Ansätze (Beladung mit Metallionen) wurden je 0,3 M NiCl₂-, 0,3 M CuSO₄- oder 0,3 M ZnCl₂-Lösungen verwendet. Es wurden wäßrige, ungepufferte Metallsalzlösungen verwendet. 200 µl der Zell-Lysatüberstände wurden auf jede dieser Minisäulen gegeben. Die Säulen wurden anschließend zweimal mit 250 µl Äquilibrierungspuffer gewaschen. Die gebundenen Proteine wurden mit zweimal 100 µl 0,5 M Imidazol in Äquilibrierungspuffer eluiert. Die Chelatsepharose in den Filtern kann mit 250 µl 50 mM EDTA, 1 M NaCl in Wasser regeneriert werden und für weitere Screeningversuche verwendet werden.

### Beispiel 5: IMAC-Versuche

Für den Versuch wurde ein üblicher Chromatographieaufbau aus einer Glassäule, zwei peristaltischen Pumpen zum Aufbringen der Lösungen, einem UV-Detektor (LKB UV-MII), einem Drucker (LKB RIC 102) und einem Fraktionssammler (LKB FRAC-200) gewählt. Alle Geräte stammten von der Firma Pharmacia. Die Säule wurde mit chelatisierender Sepharose Fast-Flow Gel (Pharmacia) gefüllt, mit 7 Bettvolumen deinosiertem Wasser gewaschen und mit 7 Bettvolumen 0,3 M NiCl₂-Lösung mit den Metallionen beladen. Anschließend wurde die Säule mit 7 Bettvolumen IMAC-Puffer (50 mM NaPhosphat, pH 8,0, 250 mM NaCl) gewaschen und äquilibriert. 1 ml Proben der Zell-Lysate wurden auf die Säule mit einer Flußrate von 1,5 ml/min aufgebracht und mit 10 Bettvolumen IMAC-Puffer gewaschen. Die Elution der gebundenen Proteine erfolgt über einen ansteigenden Gradienten mit 0,5% einer 0,5 M Imidazollösung pro ml Elutionslösung und schließlich 5 Bettvolumen einer 0,5 M Imidazol/Wasserlösung, Die Proteinfraktionen wurden über UV-Detektion ermittelt und gesammelt. Nach Abschluß wurde die Säule mit 50 mM EDTA/1 M NaCl-Lösung gewaschen und regeneriert. Dieser Waschschritt löst das Metall, gebundene Zellreste und Proteine von der Säule. Die eluierten Fraktionen wurden sowohl optisch als auch spektroskopisch untersucht. Einige der untersuchten Klone zeigten keine Affinität zur Matrix (siehe Tab.I, M15, M16) während andere eine gute Bindung aufwiesen (siehe Tab.I, M13, Z5 und Z7). Die Klone M13 und Z5 eluierten in einer scharfen Bande von der Säule, während der Klon Z7 auf der Säule schmierte.

### Beispiel 6: Vergleichsversuch mit egfp-Wildtyp und his-tag

Der Klon M13 wurde in einem Vergleichsversuch mit dem egfp-Wildtypprotein und den üblichen his-tags verglichen. Das egfp-Wildtypprotein bindet nicht an die Metallchelatsäulen. Nach dem Waschen der Säule war keine Fluoreszenz mehr auf der Säule detektierbar. Der Klon M13 bindet in einer scharfen Bande auf der Säule während die his-tag-Proteine über die gesamte Säule binden. Dies ist auf einer geringere Affinität zurückzuführen, die letztlich zu einer geringeren Kapazität der Säule führt. Die Proteinausbeute liegt im Falle von M13 mit 56% höher als mit den his-tags mit 48%.

**Tabelle I: Sindungsversuche an Ni-Metallchelatsäulen**

| Klon | Aminosäuresequenz | | | | | | | | | | |
|---|---|---|---|---|---|---|---|---|---|---|---|
| A6 | His | Gln | His | Glu | Gly | Arg | Cys | Lys | Glu | Cys | gfp |
| A8 | His | Cys | His | Pro | Glu | Leu | Cys | Stop | Leu | Cys | gfp |
| A13 | His | Leu | His | Ser | Ile | Gly | Cys | Pro | Stop | Cys | gfp |
| M13 | His | Asn | His | Arg | Tyr | Gly | Cys | Gly | Cys | Cys | gfp |
| M14 | His | Ser | His | Ser | Val | Gly | Cys | Phe | Phe | Cys | gfp |
| M15 | His | Gly | His, | Thr | Leu | Ser | Cys | Gly | Leu | Cys | gfp |
| M16 | His | Ser | His | Thr | Leu | Arg | Cys | Lys | Gly | Cys | gfp |
| M16a | His | Ser | His | Stop | Leu | Arg | Cys | Lys | Gly | Cys | gfp |
| Z4 | His | Stop | His | Asn | Stop | Val | Cys | Ala | Thr | Cys | gfp |
| Z5 | His | Arg | His | Gly | Thr | Asn | Cys | Leu | Lys | Cys | gfp |
| Z7 | His | Ile | His | Gln | Ser | Asn | Cys | Gln | Val | Cys | gfp |
| Z11 | His | Thr | His | Ala | Ser | Gly | Cys | Stop | Stop | Cys | gfp |
| Z13 | His | Cys | His | Thr | Trp | Cys | Cys | Asn | Stop | Cys | gfp |

Die Klone A6, A10, M13, Z5 und Z7 binden gut an die Metallchelatsäule, während die Klone M14, M15 und M16 keine Bindung zeigten.

**Tabelle II: Weitere sequenzierte im High-Throughput-Screening durch Fluoreszenz aufgefallene Klone**

| Klon | Aminosäuresequenz | | | | | | | | | | |
|---|---|---|---|---|---|---|---|---|---|---|---|
| A1 | His | Gly | His | Met | Glu | Arg | Cys | Leu | Val | Cys | gfp |
| A2 | His | Lys | His | Ala | Arg | Ser | Cys | Met | Gly | Cys | gfp |
| A3 | His | Phe | His | Thr | Val | Phe | Cys | Phe | Ser | Cys | gfp |
| A4 | His | Arg | His | Arg | Gly | Met | Cys | Thr | Ala | Cys | gfp |
| A12 | His | Asp | His | Arg | Gly | Val | Cys | Gly | Leu | Cys | gfp |
| A14 | His | Asp | His | Glu | Arg | Leu | Cys | His | Asn | Cys | gfp |
| X8 | His | Gly | His | Gly | Asn | Arg | Cys | Cys | Gly | Cys | gfp |
| X9 | His | Arg | His | Gly | Thr | Ala | Cys | Met | Asp | Cys | gfp |
| X11 | His | Ile | His | Ile | Met | Thr | Cys | Leu | Ser | Cys | gfp |
| X12 | His | Thr | His | Pro | Arg | Ser | Cys | Ala | Glu | Cys | gfp |
| X15 | His | Gly | His | Asp | Arg | Thr | Cys | Arg | Gly | Cys | gfp |
| X16 | His | Arg | His | Ala | Ile | Ser | Cys | Ile | Gly | Cys | gfp |
| X17 | His | Ile | His | Arg | Gly | Asp | Cys | Tyr | Glu | Cys | gfp |
| X18 | His | His | His | Gly | Ser | Thr | Cys | Pro | Thr | Cys | gfp |
| X19 | His | His | His | Phe | His | Ser | Cys | Phe | Tyr | Cys | gfp |
| Z8 | His | Lys | His | Val | Asp | His | Cys | Gly | Arg | Cys | gfp |
| Z9 | His | Ser | His | Leu | Thr | Leu | Cys | Leu | Gly | Cys | gfp |
| Z10 | His | Thr | His | Gln | Ser | Gln | Cys | Gly | Arg | Cys | gfp |
| Z14 | His | Arg | His | Leu | Phe | Trp | Cys | Ser | Glu | Cys | gfp |

### Beispiel 7: Metallbindungsaffinität

Viele der Klone zeigten eine bevorzugte Bindung an Ni²⁺ oder Cu²⁻. Im Falle von M13 konnte keine Bindung an Zn³⁺ beobachtet werden. Bei Verwendung von Ni-Chelatsäulen zeigt der Klon M13 eine deutlich bessere Reinigung der Proteine im Vergleich zu den his-tags. Diese führten umgekehrt bei Verwendung von Cu-Chelatsäulen zu einem reineren Produkt im Vergleich zu M13, wobei jedoch, da die Bindung an das Säulenmaterial in beiden Fällen sehr stark ist, durch die drastischen Elutionsbedingungen Cu-Ionen ausgewaschen wurden. Dies führt zu Produktverunreinigungen.

### Beispiel 8: Vergleichsversuch zwischen Sequenz der ATPase-439 und erfindungsgemäßen Proteinfragmenten

Der Vergleichsklon der ATPase-439 wurde analog Beispiel 1 und 6 durchgeführt. Als Primer wurde folgender Primer 5'-GCAATACCATGGGGCATATTCATAATCTTGATTGTCCTGATTGT-3' verwendet. Die anderen Primer und die PCR-Bedingungen waren wie unter Beispiel 1 beschrieben.

Der Versuch wurde mit dem Quiagen Ni-NTA Spin Kit unter nativen Bedingungen durchgeführt, unter diesen Bedingungen wurde wie beschrieben lysiert; die bereits fertig beladenen Säulen wurden mit 600 µl 50 mM Na-Phosphatpuffer, pH 8,0, 300 mM NaCl äquilibriert und 2 min bei 2000 rpm (= 420 x g) zentrifugiert. Anschließend wurden 600 µl des Lysats aufgebracht und wie 2 min zentrifugiert. Mit je 600 µl 50 mM Na-Phosphatpuffer, pH 8,0, 300 mM NaCl wurde zweimal gewaschen. Danach wurde mit 600 µl 50 mM Na-Phosphatpuffer, pH 8,0, 300 mM NaCl, 0,5 M Imidazol wurde zweimal eluiert. Die Ausbeute an reinem Protein war mit dem Klon M13 1,5fach höher als der Metallbindungstelle des Vergleichsklons der ATPase-439. Klon M13 bindet damit besser und läßt sich auch besser eluieren.
## Patentansprüche

1. Peptidfragment mit der allgemeinen Sequenz
His-X¹-His-X²-X³-X⁴-Cys-X⁵-X⁶-Cys,
wobei die Variablen X¹ bis X⁶ in der Sequenz unabhängig voneinander folgende Bedeutung haben:
X¹ = eine Aminosäure ausgewählt aus der Gruppe Ala, Val, Phe, Ser, Met, Trp, Tyr, Asn, Asp oder Lys;
X² = eine Aminosäure ausgewählt aus der Gruppe Val, Ile, Phe, Pro, Trp, Tyr, Gln, Glu oder Arg;
X³ = eine Aminosäure ausgewählt aus der Gruppe Gly, Ile, Thr, Met, Trp, Tyr, Asn, Gln, Asp, Glu, Lys, Arg oder His;
X⁴ = eine Aminosäure ausgewählt aus der Gruppe Val, Phe, Pro, Cys, Met, Trp, Asn, Glu, Arg oder His;
X⁵ = eine Aminosäure ausgewählt aus der Gruppe Gly, Ser, Cys, Met, Trp, Asn, Glu, Lys oder Arg;
X⁶ = eine Aminosäure ausgewählt aus der Gruppe Phe, Pro, Ser, Cys, Trp, Tyr oder Gln.

2. Peptidfragment nach Anspruch 1, in der die Variablen X¹ bis X⁸ in der Sequenz unabhängig voneinander folgende Bedeutung haben:
X¹ = eine Aminosäure ausgewählt aus der Gruppe Phe, Ser, Asn, Asp oder Lys;
X² = eine Aminosäure ausgewählt aus der Gruppe Val, lle, Phe, Pro, Gln, Glu oder Arg;
X³= eine Aminosäure ausgewählt aus der Gruppe Gly, Ile, Thr, Met, Trp, Tyr, Asn, Asp, Glu, Arg oder His;
X⁴= eine Aminosäure ausgewählt aus der Gruppe Val, Phe, Cys, Met, Trp, Asn, Arg oder His;
X⁵ = eine Aminosäure ausgewählt aus der Gruppe Gly, Ser, Cys, Met, Asn, Glu, Lys oder Arg;
X⁶= eine Aminosäure ausgewählt aus der Gruppe Phe, Ser, Cys, oder Tyr.

3. Peptidfragment nach Anspruch 1 oder 2, in der die Variablen X¹ bis X⁶ in der Sequenz unabhängig voneinander folgende Bedeutung haben:
X¹ = Asn;
X² = Gln, Glu oder Arg;
X³ = Gly, Thr oder Tyr;
X⁴ = Asn oder Arg;
X⁵= Gly oder Lys;
X⁶ = Cys.

4. Peptidfragmente mit den Sequenzen
His-Gln-His-Glu-Gly-Arg-Cys-Lys-Glu-Cys
His-Asn-His-Arg-Tyr-Gly-Cys-Gly-Cys-Cys
His-Arg-His-Gly-Thr-Asn-Cys-Leu-Lys-Cys
His-Ile-His-Gln-Ser-Asn-Cys-Gln-Val-Cys.

5. Fusionsprotein enthaltend ein Proteinfragment gemäß den Ansprüchen 1 bis 3.

6. Nukleinsäurefragment kodierend für ein Proteinfragment gemäß den Ansprüchen 1 bis 3.

7. Nukleinsäuren enthaltend ein Nukleinsäurefragment gemäß Anspruch 6.

8. Nukleinsäuren kodierend für ein Fusionsprotein gemäß Anspruch 5.

9. Vektor enthaltend ein Nukleinsäurefragment gemäß Anspruch 6 oder 8.

10. Verfahren zur Herstellung von Fusionsproteinen gemäß Anspruch 5, **dadurch gekennzeichnet, daß** man ein Nukleinsäurefragment gemäß Anspruch 6 mit einem Gen, das für ein Protein kodiert, fusioniert.

11. Verfahren zur Reinigung von Fusionsproteinen gemäß Anspruch 5, **dadurch gekennzeichnet, daß** man
a) Flüssigkeiten, die das Fusionsprotein enthalten, so mit immobilisierten Metallionen in Kontakt bringt, daß sich zwischen den Metallionen und dem Fusionsprotein eine Affinitätsbindung ausbilden kann,
b) Entfernen nicht gebundener in der Flüssigkeit enthaltener Substanzen,
c) Eluieren des gebundenen Fusionsproteins, in dem die Affinitätsbindung durch Änderung des Flüssigkeits milieus aufgehoben wird und
d) Sammlung des gereinigten Fusionsproteins.

12. Verwendung eines Proteinfragments gemäß den Ansprüchen 1 bis 3 oder eines Nukleinsäurefragments gemäß Anspruch 6 zur Reinigung von Proteinen .

13. Verfahren zum Screening auf Proteinfragmente die in der Lage sind mit immobilisierten Metallionen eine reversible Affinitätsbindung einzugehen, **dadurch gekennzeichnet, daß** man folgende Schritte durchführt:
a) Herstellung einer Nukleinsäurebibliothek ausgehend von einer beliebigen Nukleinsäuresequenz, die für ein Proteinfragment der Sequenz
His-X¹-His-X²-X³-X⁴-Cys-X⁵-X⁶-Cys,
kodiert, wobei die Histidin- und Cysteinreste der Sequenz in der Nukleinsäurebibliothek konserviert werden,
b) Fusion der Nukleinsäuren der Bibliothek an ein Reportergen, das die Detektion des durch die erhaltene Nukleinsäure kodierten Fusionsproteins über seine Bindung an die immobilisierten Metallionen ermöglicht und
c) Auswahl der Nukleinsäuresequenzen, die eine gegenüber der in der natürlichen Helicobacter pilori ATPase-439 Sequenz um mindestens 1,5 fach stärkere reversible Bindung an die immobilisierten Metallionen aufweisen.

14. Verfahren nach Anspruch 13, **dadurch gekennzeichnet**, das als Reportergen das egf-Protein von Aequoria victoria verwendet wird.

15. Verfahren zur Detektion von Proteinen, **dadurch gekennzeichnet, daß** man aus einer Proteinmischung einzelne Proteine, die ein Proteinfragment gemäß Anspruch 1 enthalten, über Antikörper nachweist, die gegen das Proteinfragment gerichtet sind.

16. Verwendung eines Proteinfragments gemäß den Ansprüchen 1 bis 3 oder eines Nukleinsäurefragments gemäß Anspruch 6 zur Reinigung von Proteinen.

## Claims

1. A peptide fragment having the general sequence
His-X¹-His-X²-X³-X⁴-Cys-X⁵-X⁶-Cys,
where the variables X¹ to X⁶ in the sequence have the following meanings independently of one another:
X¹ = an amino acid selected from the group of Ala, Val, Phe, Ser, Met, Trp, Tyr, Asn, Asp or Lys;
X² = an amino acid selected from the group of Val, Ile, Phe, Pro, Trp, Tyr, Gln, Glu or Arg;
X³ = an amino acid selected from the group of Gly, Ile, Thr, Met, Trp, Tyr, Asn, Gln, Asp, Glu, Lys, Arg or His;
X⁴ = an amino acid selected from the group of Val, Phe, Pro, Cys, Met, Trp, Asn, Glu, Arg or His;
X⁵ = an amino acid selected from the group of Gly, Ser, Cys, Met, Trp, Asn, Glu, Lys or Arg;
X⁶ = an amino acid selected from the group of Phe, Pro, Ser, Cys, Trp, Tyr or Gln;

2. The peptide fragment according to claim 1, in which the variables X¹ to X⁶ in the sequence have the following meanings independently of one another:
X¹ = an amino acid selected from the group of Phe, Ser, Asn, Asp or Lys;
X² = an amino acid selected from the group of Val, Ile, Phe, Pro, Gln, Glu or Arg;
X³ = an amino acid selected from the group of Gly, Ile, Thr, Met, Trp, Tyr, Asn, Asp, Glu, Arg or His;
X⁴ = an amino acid selected from the group of Val, Phe, Cys, Met, Trp, Asn, Arg or His;
X⁵ = an amino acid selected from the group of Gly, Ser, Cys, Met, Asn, Glu, Lys or Arg;
X⁶ = an amino acid selected from the group of Phe, Ser, Cys, or Tyr.

3. The peptide fragment according to claim 1 or 2, in which the variables X¹ to X⁶ in the sequence have the following meanings independently of one another:
X¹ = Asn;
X² = Gln, Glu or Arg;
X³ = Gly, Thr or Tyr;
X⁴ = Asn or Arg;
X⁵ = Gly or Lys;
X⁶ = Cys.

4. Peptide fragments having the sequences
His-Gln-His-Glu-Gly-Arg-Cys-Lys-Glu-Cys
His-Asn-His-Arg-Tyr-Gly-Cys-Gly-Cys-Cys
His-Arg-His-Gly-Thr-Asn-Cys-Leu-Lys-Cys
His-Ile-His-Gln-Ser-Asn-Cys-Gln-Val-Cys.

5. A fusion protein comprising a protein fragment according to claims 1 to 3.

6. A nucleic acid fragment coding for a protein fragment according to claims 1 to 3.

7. A nucleic acid comprising a nucleic acid fragment according to claim 6.

8. A nucleic acid coding for a fusion protein according to claim 5.

9. A vector comprising a nucleic acid fragment according to claim 6 or 8.

10. A process for preparing fusion proteins according to claim 5, which comprises fusing a nucleic acid fragment according to claim 6 to a gene which codes for a protein.

11. The process for purifying fusion proteins according to claim 5, which comprises
a) bringing liquids which comprise the fusion protein into contact with immobilized metal ions in such a way that an affinity linkage can form between the metal ions and the fusion protein,
b) removing unbound substances present in the liquid,
c) eluting the bound fusion protein in which [sic] the affinity linkage is abolished by changing the liquid medium and
d) collecting the purified fusion protein.

12. The use of a protein fragment according to claims 1 to 3 or of a nucleic acid fragment according to claim 6 for purifying proteins.

13. A process for screening protein fragments able to enter into a reversible affinity linkage with immobilized metal ions, which comprises carrying out the following steps:
a) preparing a nucleic acid library starting from any suitable nucleic acid sequence which codes for a protein fragment of the sequence
Hi_{S}-X¹-His-X²-X³-X⁴-Cys-X⁵-X⁶-Cys,
where the histidine and cysteine residues of the sequence are conserved in the nucleic acid library,
b) fusing the nucleic acids of the library to a reporter gene which makes it possible to detect the fusion protein encoded by the resulting nucleic acid via its binding to the immobilized metal ions and
c) selecting the nucleic acid sequences which display a reversible binding to the immobilized metal ions which is at least 1.5 times stronger than the sequence in the natural Helicobacter pilori [sic] ATPase-439.

14. The process according to claim 13, wherein the egf protein from Aequoria victoria is used as reporter gene.

15. A method for detecting proteins, which comprises detecting individual proteins which comprise a protein fragment according to claim 1 in a protein mixture via antibodies which are directed against the protein fragment.

16. The use of a protein fragment according to claims 1 to 3 or of a nucleic acid fragment according to claim 6 for purifying proteins.

## Revendications

1. Fragment peptidique présentant la séquence générale :
His-X¹-His-X¹-X³-X⁴-Cys-X⁵-X⁶-cys,
où les variables X¹ à X⁶ ont dans la séquence, indépendamment l'une de l'autre, la signification suivante :
X¹ = un acide aminé choisi parmi le groupe Ala, Val, Phe, Ser, Met, Trp, Tyr, Asn, Asp ou Lys,
X² = un acide aminé choisi parmi le groupe Val, Ile, Phe, Pro, Trp, Tyr, Gln, Glu ou Arg,
X³ = un acide aminé choisi parmi le groupe Gly, Ile, Thr, Met, Trp, Tyr, Asn, Gln, Asp, Glu, Lys, Arg ou His,
X⁴ = un acide aminé choisi parmi le groupe Val, Phe, Pro, Cys, Met, Trp, Asn, Glu, Arg ou His,
X⁵ = un acide aminé choisi parmi le groupe Gly, Ser, Cys, Met, Trp, Asn, Glu, Lys ou Arg,
X⁶ = un acide aminé choisi parmi le groupe Phe, Pro, Ser, Cys, Trp, Tyr ou Gln.

2. Fragment peptidique suivant la revendication 1, dans lequel les variables X¹ à X⁶ ont dans la séquence, indépendamment l'une de l'autre, la signification suivante :
X¹ = un acide aminé choisi parmi le groupe Phe, Ser, Asn, Asp ou Lys,
X² = un acide aminé choisi parmi le groupe Val, Ile, Phe, Pro, Gln, Glu ou Arg,
X³ = un acide aminé choisi parmi le groupe Gly, Ile, Thr, Met, Trp, Tyr, Asn, Asp, Glu, Arg ou His,
X⁴ = un acide aminé choisi parmi le groupe Val, Phe, Cys, Met, Trp, Asn, Arg ou His,
X⁵ = un acide aminé choisi parmi le groupe Gly, Ser, Cys, Met, Asn, Glu, Lys ou Arg,
X⁶ = un acide aminé choisi parmi le groupe Phe, Ser, Cys ou Tyr.

3. Fragment peptidique suivant la revendication 1 ou 2, dans lequel les variables X¹ à X⁶ ont dans la séquence, indépendamment l'une de l'autre, la signification suivante :
X¹ = Asn;
X² = Gln, Glu ou Arg;
X³ = Gly, Thr ou Tyr;
X⁴ = Asn ou Arg;
X⁵ = Gly ou Lys;
X⁶ = Cys.

4. Fragments peptidiques présentant les séquences :
His-Gln-His-Glu-Gly-Arg-Cys-Lys-Glu-Cys
His-Asn-His-Arg-Tyr-Gly-Cys-Gly-Cys-Cys
His-Arg-His-Gly-Thr-Asn-Cys-Leu-Lys-Cys
His-Ile-His-Gln-Ser-Asn-Cys-Gln-Val-Cys.

5. Protéine de fusion contenant un fragment de protéine suivant les revendications 1 à 3.

6. Fragment d'acide nucléique codant pour un fragment de protéine suivant les revendications 1 à 3.

7. Acides nucléiques contenant un fragment d'acide nucléique suivant la revendication 6.

8. Acides nucléiques codant pour une protéine de fusion suivant la revendication 5.

9. Vecteur contenant un fragment d'acide nucléique suivant la revendication 6 ou 8.

10. Procédé de préparation de protéines de fusion suivant la revendication 5, **caractérisé en ce qu'**on fusionne un fragment d'acide nucléique suivant la revendication 6 avec un gène qui code pour une protéine.

11. Procédé de purification de protéines de fusion suivant la revendication 5, **caractérisé en ce que**
a) on met en contact des liquides, qui contiennent la protéine de fusion, avec des ions métalliques immobilisés de façon à pouvoir réaliser une liaison par affinité entre les ions métalliques et la protéine de fusion,
b) on élimine les substances non liées, contenues dans le liquide,
c) on élue la protéine de fusion fixée, dans laquelle la liaison par affinité est rompue par modification du milieu liquide, et
d) on récolte la protéine de fusion purifiée.

12. Utilisation d'un fragment de protéine suivant les revendications 1 à 3 ou d'un fragment d'acide nucléique suivant la revendication 6, pour la purification de protéines.

13. Procédé de criblage de fragments de protéine, qui sont en état de donner naissance à une liaison par affinité réversible avec des ions métalliques immobilisés, **caractérisé en ce qu'**on effectue les étapes suivantes :
a) une préparation d'une banque d'acides nucléiques en partant d'une séquence d'acides nucléiques quelconque qui code pour un fragment de protéine de la séquence :
His-X¹-His-X²-X³-X⁴Cys-X⁵-X⁶-cys,
où les radicaux d'histidine et de cystéine de la séquence sont conservés dans la banque d'acides nucléiques,
b) une fusion des acides nucléiques de la banque sur un gène rapporteur, qui permet la détection de la protéine de fusion codée par l'acide nucléique obtenu par l'intermédiaire de sa liaison sur les ions métalliques immobilisés, et
c) une sélection des séquences d'acides nucléiques qui présentent une liaison réversible sur les ions métalliques immobilisés au moins 1,5 fois plus forte que la séquence ATPase-439 d'Helicobacter pilori naturel.

14. Procédé suivant la revendication 13, **caractérisé en ce que**, comme gène rapporteur, on utilise la protéine egf d'Aequoria victoria.

15. Procédé de détection de protéines, **caractérisé en ce qu'**on décèle dans un mélange de protéines des protéines isolées qui contiennent un fragment de protéine suivant la revendication 1, par l'intermédiaire d'anticorps qui sont dirigés contre le fragment de protéine.

16. Utilisation d'un fragment de protéine suivant les revendications 1 à 3 ou d'un fragment d'acide nucléique suivant la revendication 6, pour la purification de protéines.


<110> BASF Aktiengesellschaft
<120> Neue Peptidfragmente zur Reinigung von Proteinen
<130> PCT/EP99/03469
<140> PCT/EP99/03469
   <141> 1999-05-20
<150> DE 198 22 823.6
   <151> 1998-05-20
<160> 5
<170> PatentIn Vers. 2.0
<210> 1
   <211> 10
   <212> PRT
   <213> Artificial Sequence
<220>
   <223> Sequenz zum Aufreinigen von Proteinen
<400> 1
   His Xaa His Xaa Xaa Xaa Cys Xaa Xaa Cys
    1               5                   10
<210> 2
   <211> 10
   <212> PRT
   <213> Artificial Sequence
<220>
   <223> Sequenz zum Aufreinigen von Proteinen
<400> 2
   His Gln His Glu Gly Arg Cys Lys Glu Cys
    1               5                   10
<210> 3
   <211> 10
   <212> PRT
   <213> Artificial Sequence
<220>
   <223> Sequenz zum Aufreinigen von Proteinen
<400> 3
   His Asn His Arg Tyr Gly Cys Gly Cys Cys
    1               5                   10
<210> 4
   <211> 10
   <212> PRT
   <213> Artificial Sequence
<220>
   <223> Sequenz zum Aufreinigen von Proteinen
<400> 4
   His Arg His Gly Thr Asn Cys Leu Lys Cys
    1               5                   10
<210> 5
   <211> 10
   <212> PRT
   <213> Artificial Sequence
<220>
   <223> Sequenz zum Aufreinigen von Proteinen
<400> 5
   His Ile His Gln Ser Asn Cys Gln Val Cys
    1               5                  10